# EUROPÄISCHE PATENTSCHRIFT

(11) **EP 0 668 292 B1**
(45) Veröffentlichungstag und Bekanntmachung des Hinweises auf die Patenterteilung: **13.05.1998**
(21) Anmeldenummer: 95101748.2
(22) Anmeldetag: 09.02.1995
(51) Int. Cl.: C07K 14/62, C07K 1/113

(54) **Verfahren zur Gewinnung von Insulin mit korrekt verbundenen Cystinbrücken**
Process to obtain insulin with correct cystin bridges
Procédé pour obtenir l'insuline avec les ponts cystiniques corrects

(30) Priorität: 18.02.1994 DE 4405179
(43) Veröffentlichungstag der Anmeldung: 23.08.1995
(73) Patentinhaber: HOECHST AKTIENGESELLSCHAFT, 65929 Frankfurt am Main (DE)
(72) Erfinder: Obermeier, Rainer, Dr., D-65795 Hattersheim (DE); Gerl, Martin, Dr., D-65830 Kriftel (DE); Ludwig, Jürgen, D-63636 Brachttal (DE); Sabel, Walter, D-65520 Bad Camberg (DE)

(56) Entgegenhaltungen:
- EP-A- 0 037 255
- EP-A- 0 379 162
- EP-A- 0 600 372
- PROC. 7TH AM. PEPT. SYMP., 1981, Seiten 729-738; FRANK, B.H. ET AL.: 'The production of human proinsulin and its transformation to human insulin and C-peptide'
- GENE, Bd. 16, 1981, Seiten 63-71; WETZEL, R.: 'Expression in Escherichia coli of a chemically synthesized gene for a "mini-C" analog of human proinsulin'

## Beschreibung

Humaninsulin ist ein Protein mit zwei Aminosäureketten mit zusammen 51 Aminosäureresten. In den beiden Aminosäureketten kommen 6 Cysteinreste vor, wobei je zwei Cysteinreste über eine Disulfidbrücke untereinander verbunden sind. In biologisch aktivem Humaninsulin sind die A- und B-Ketten über zwei Cystinbrücken miteinander verbunden, und eine weitere Cystinbrücke kommt in der A-Kette vor. Innerhalb eines Humaninsulinmoleküls gibt es statistisch gesehen 15 Möglichkeiten zur Ausbildung von Disulfidbrücken. Im biologisch wirksamen Humaninsulin kommt nur eine der 15 Möglichkeiten vor. Die folgenden Cysteinreste sind im Humaninsulin miteinander verknüpft:
A 6 - A 11
A 7 - B 7
A 20 - B 19
Die Buchstaben A und B stehen für die jeweilige Insulinaminosäurekette, die Zahl für die Position des Aminosäurerestes, die vom Amino- zum Carboxylende der jeweiligen Aminosäurekette gezählt wird. Disulfidbrücken können auch zwischen zwei Humaninsulinmolekülen ausgebildet werden, so daß leicht unübersehbar viele verschiedene Disulfidbrücken entstehen können.

Ein bekanntes Verfahren zur Herstellung von Humaninsulin beruht auf der Verwendung von humanem Proinsulin. Humanes Proinsulin ist ein Protein mit einer linearen Aminosäurekette aus 86 Aminosäureresten, wobei die B- und A-Ketten des Humaninsulins über ein C-Peptid mit 35 Aminosäureresten miteinander verbunden sind. Die Ausbildung der in Humaninsulin vorkommenden Disulfidbrücken erfolgt über ein Zwischenprodukt, wobei die Cysteinreste des Humaninsulins mit einer Schwefelschutzgruppe, z.B. einer S-Sulfonat (-S-SO₃⁻)-Gruppe versehen sind (EP 0 037 255). Ferner ist ein Verfahren zur Gewinnung von Proinsulin mit korrekt verbundenen Cystinbrücken bekannt (Biochemistry, 60, (1968), Seiten 622 bis 629), das von Proinsulin gewonnen aus Schweinepankreas ausgeht, bei dem die Cysteinreste als Thiolreste (-SH) vorliegen. Unter dem Begriff "korrekt verbundenen Cystinbrücken" versteht man die Disulfidbrücken, die in biologisch aktivem Insulin aus Säugetieren vorkommen.

Gentechnische Verfahren erlauben es, menschliches Proinsulin oder Proinsulin, das eine von Humaninsulin abweichende Aminosäuresequenz und/oder Aminosäurekettenlänge hat, in Mikroorganismen herzustellen. Die von gentechnisch veränderten Escherichia-coli-Zellen hergestellten Proinsuline haben keine korrekt verbundenen Cystinbrücken. Ein Verfahren zur Gewinnung von Humaninsulin mit E. coli (EP 0 055 945) beruht auf folgenden Verfahrensschritten:
Fermentation der Mikroorganismen - Zellaufschluß - Isolierung des Fusionproteins - Bromcyanspaltung des Fusionproteins - Isolierung des Spaltprodukts mit der Proinsulinsequenz - Schutz der Cysteinreste von Proinsulin durch S - Sulfonat-Gruppen - Ausbildung der korrekt verbundenen Cystinbrücken - Sulfitolyse - Entsalzen des Proinsulins - Chromatographische Reinigung des Proinsulins mit korrekt verbundenen Cystinbrücken - Konzentrierung der Proinsulinlösung - Chromatographische Reinigung der konzentrierten Proinsulinlösung - Enzymatische Spaltung des Proinsulins zur Gewinnung von Humaninsulin - Chromatographische Reinigung des entstandenen Humaninsulins.

Nachteile dieses Verfahrens sind die Anzahl der Verfahrensschritte und die Verluste bei den Reinigungsschritten, die zu einer geringen Ausbeute an Insulin führen. Wegen des vielstufigen Verfahrensweges müssen erhebliche Verluste in Kauf genommen werden. Von der Stufe des isolierten Fusionproteins über Bromcyanspaltung, Sulfitolyse und Reinigung des Proinsulins muß mit einem bis zu 40%igem Verlust von Proinsulin gerechnet werden (EP 0 055 945). Ähnlich hohe Verluste können im Verlauf der nachfolgenden Reinigungsschritte bis zum Endprodukt auftreten.

Ausbeutesteigerungen bei der gentechnischen Herstellung von Humaninsulin oder Insulinderivaten lassen sich dann erzielen, wenn die Zahl der notwendigen Verfahrensschritte wesentlich verringert werden kann.

Aufgabe der vorliegenden Erfindung war es, ein Verfahren zur Gewinnung von Insulin mit korrekt verbundenen Cystinbrücken der Insulinaminosäurekette zu entwickeln, bei dem weniger Verfahrensschritte nötig sind und insgesamt geringere Reinigungsverluste auftreten.

Es wurde nun ein Verfahren zur Gewinnung von Insulin der Formel I 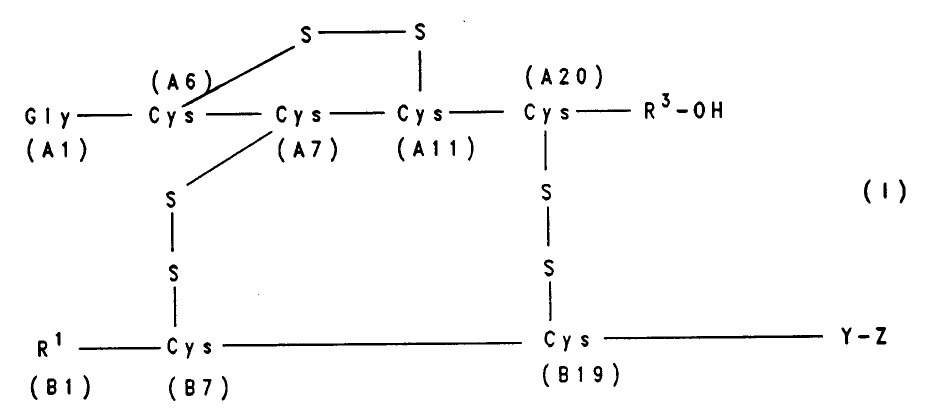 gefunden,
das dadurch gekennzeichnet ist, daß man
A) ein Protein der Formel II,

   R²-R¹-B2-B29-Y-X-Gly-A2-A20-R³ (II)

   mit einer Menge eines Mercaptans, die 2 bis 10 Reste -SH des Mercaptans pro Cysteinrest des Proteins der Formel II ergibt, in Gegenwart von mindestens einem chaotropen Hilfsstoff in einem wäßrigen Medium bei einem pH-Wert von 10 bis 11 und einer Konzentration des Proteins der Formel II von 0,05 bis 0,3 g pro Liter wäßrigen Mediums umsetzt und
B) das erhaltene Proinsulin mit korrekt verbundenen Cystinbrücken mit Trypsin, einem trypsinähnlichen Enzym und gegebenfalls zusätzlich mit Carboxypeptidase B oder einer Mischung der genannten Enzyme zu dem Insulin der Formel I mit korrekt verbundenen Cystinbrücken umsetzt,
C) das so erhaltene Umsetzungsprodukt mit 3 bis 50 g eines hydrophoben Adsorberharzes pro Liter wäßrigen Mediums bei einem pH-Wert von 4 bis 7 versetzt,
D) das Adsorberharz mit adsorbiertem Insulin der Formel I isoliert und
E) das Insulin der Formel I vom Adsorberharz desorbiert; dabei ist in Formeln I und II
   - X: a) ein Aminosäurerest aus der Gruppe Lys oder Arg oder
   b) ein Peptid mit 2 bis 35 Aminosäureresten, enthaltend den Aminosäurerest Arg oder Lys am N-terminalen- und Carboxylende des Peptids,
   - Y: ein genetisch kodierbarer Aminosäurerest,
   - Z: a) ein Aminosäurerest aus der Gruppe Arg oder Lys,
   b) ein Peptid mit 2 oder 3 Aminosäureresten, enthaltend den Aminosäurerest Arg oder Lys am Carboxylende des Peptids, oder
   c) OH,
   - R¹: ein Phenylalaninrest oder eine kovalente Bindung,
   - R²: a) ein Wasserstoffatom,
   b) ein Aminosäurerest aus der Gruppe Lys oder Arg oder
   c) ein Peptid mit 2 bis 45 Aminosäureresten, enthaltend den Aminosäurerest Lys oder Arg am Carboxylende des Peptids,
   - R³: ein genetisch kodierbarer Aminosäurerest und
   die Reste A2 - A20 entsprechen der Aminosäuresequenz der A-Kette von Humaninsulin, tierischen Insulin oder einem Insulinderivat und die Reste B2 - B 29 entsprechen der Aminosäuresequenz der B-Kette von Humaninsulin, tierischen Insulin oder einem Insulinderivat.

Bevorzugt wird ein Protein der Formel II eingesetzt; dabei ist
- X: der Aminosäurerest Arg oder ein Peptid mit der Aminosäuresequenz der C-Kette von Humaninsulin,
- Y: ein Aminosäurerest aus der Gruppe Ala, Thr oder Ser,
- R¹: der Aminosäurerest Phe,
- R²: a) Wasserstoffatom oder
b) ein Peptid mit 2 bis 25 Aminosäureresten, enthaltend Arg am Carboxylende des Peptids,
- R³: ein Aminosäurerest aus der Gruppe Asn, Ser oder Gly,
und die Reste A2 - A20 und B2 - B29 entsprechen der Aminosäuresequenz der B- und A-Ketten von Humaninsulin und
es entsteht ein Insulin der Formel I mit korrekt verbundenen Cystinbrücken, dabei haben Y, R¹, R², R³, A2-A20 und B2-B29 die obengenannte Definition und
- Z: ist der Aminosäurerest Arg, der Peptidrest Arg-Arg oder OH.

Besonders bevorzugt wird ein Protein der Formel II eingesetzt; dabei ist
- X: der Aminosäurerest Arg oder ein Peptid mit 2 bis 35 Aminosäurereste, wobei am Anfang und Ende des Peptids zwei basische Aminosäurereste, insbesondere Arg und/oder Lys stehen,
- Y: der Aminosäurerest Thr,
- R¹: der Aminosäurerest Phe,
- R²: a) Wasserstoffatom oder
b) ein Peptid mit 2 bis 15 Aminosäureresten, an dessen Carboxylende der Aminosäurerest Arg steht,
- R³: der Aminosäurerest Gly oder Asn,
und die Reste A2 - A20 und B2 - B29 entsprechen der Aminosäuresequenz der A- und B-Ketten von Humaninsulin und
es entsteht ein Insulin der Formel I mit korrekt verbundenen Cystinbrücken, dabei haben Y, R¹, R², R³, A2-A20 und B2-B29 die obengenannte Definition und
- Z: ist der Aminosäurerest Arg, der Peptidrest Arg-Arg oder Lys-Lys oder OH.

Überraschenderweise wurde gefunden, daß das Protein der Formel II nicht in einem üblicherweise dem "Refolding" vorausgehenden Reaktionsschritt vollständig reduziert werden muß (R. Jaenicke, R. Rudolph, 1989, Folding Proteins, in Protein Structure a practical Approach; Ed. Creighton, T.E., Seiten 191-224, JRL Press Oxford; EP 0 219 874) und daß bei der beschriebenen Verfahrensweise trotz eines hohen Anteils von Fremdprotein (50 - 90 %) Faltungsausbeuten erzielt werden, die in vergleichbarer Höhe liegen wie bei der Faltung von entsprechend gereinigten Proinsulin mit -SH-Schutzgruppen.

Ferner wurde überraschenderweise gefunden, daß die enzymatische Umsetzung von richtig gefalteten Proinsulin zu dem Insulin der Formel I mit korrekt verbundenen Cystinbrücken auch in Gegenwart von 50 bis 90 % Fremdprotein in hoher Ausbeute möglich ist und das Insulin der Formel I mit guter Selektivität an ein hydrophobes Adsorberharz bindet.

Das erfindungsgemäße Verfahren erlaubt, verglichen mit den bisher bekannten Methoden, eine wesentlich schnellere Herstellung mit höheren Ausbeuten. Ursache dafür ist die Vermeidung der Verfahrensschritte Sulfitolyse und gegebenenfalls Bromcyanspaltung sowie die bisher unbekannte Möglichkeit, Proinsulin direkt aus ihrem denaturierten Zustand in Gegenwart begrenzter Mengen von chaotropen Salzen in hohen Ausbeuten in ein Proinsulin mit korrekt gebundenen Cystinbrücken umzusetzen und anschließend ohne weitere Reinigung enzymatisch in das Insulin der Formel I umzusetzen. Dadurch wird es möglich, das entstandene Insulin der Formel I durch Adsorption aus der Faltungslösung abzutrennen. So können die Insuline der Formel I ohne Zwischenisolierung oder Reinigung nach Desorption vom Adsorptionsmittel gewonnen werden. Das erfindungsgemäße Verfahren führt zu einer erheblich verkürzten Herstellung, die wegen kürzerer Standzeiten und Vermeidung von Verlusten Ausbeutesteigerungen in Höhe von 25-100 % gegenüber den bekannten Verfahren ermöglicht.

Die Aminosäuresequenz von Peptiden und Proteinen wird vom N-terminalen Ende der Aminosäurekette an bezeichnet. Die in Formel I in Klammern gemachten Angaben, z.B. A6, A20, B1, B7 oder B19, entsprechen der Position von Aminosäureresten in den A- oder B-Ketten des Insulins.

Für den Begriff "genetisch kodierbarer Aminosäurerest" stehen die Aminosäuren Gly, Ala, Ser, Thr, Val, Leu, Ile, Asp, Asn, Glu, Gln, Cys, Met, Arg, Lys, His, Tyr, Phe, Trp, Pro und Selenocystein.

Für die Begriffe "Reste A2 - A20" und "Reste B2 - B 29" von tierischen Insulin werden beispielsweise die Aminosäuresequenzen von Insulin aus Rindern, Schweinen oder Hühnern verstanden.Der Begriff Reste A2 - A20 und B2 - B29 von Insulinderivaten steht für die entsprechenden Aminosäuresequenzen von Humaninsulin, die durch den Austausch von Aminosäuren durch andere genetisch kodierbare Aminosäuren gebildet werden.

Der Rest Z ist immer Teil der Aminosäuresequenz von X und entsteht durch die Aktivität der Proteasen wie Trypsin, trypsinähnliches Enzym oder Carboxypeptidase B. Der Rest R³ ist der Aminosäurerest, der an Position A21 der A-Kette von Insulin steht. Der Rest Y ist der Aminosäurerest, der an Position B30 der B-Kette von Insulin steht.

Trypsin oder trypsinähnliche Enzyme sind Proteasen, die Aminosäureketten am Arginin- oder Lysinrest spalten.
Carboxypeptidase B ist eine Exoprotease, die basische Aminosäurereste wie Arg oder Lys, die am Carboxyterminalen Ende von Aminosäureketten stehen, abspaltet. (Kemmler et al., J. Biol. Chem. 246, Seiten 6786-6791).

Die A-Kette von Humaninsulin hat folgende Sequenz (Seq Id No. 1):
Gly, Ile, Val, Glu, Gln, Cys, Cys, Thr, Ser, Ile, Cys, Ser, Leu, Tyr, Gln, Leu, Glu, Asn, Tyr, Cys, Asn.

Die B-Kette von Humaninsulin hat folgende Sequenz (Seq Id No. 2 ):
Phe, Val, Asn, Gln, His, Leu, Cys, Gly, Ser, His, Leu, Val, Glu, Ala, Leu, Tyr, Leu, Val, Cys, Gly, Glu, Arg, Gly, Phe, Phe, Tyr, Thr, Pro, Lys, Thr.

Die C-Kette von Humaninsulin hat folgende Sequenz (Seq Id No. 3):
Arg, Arg, Glu, Ala, Glu, Asp, Leu, Gln, Val, Gly, Gln, Val, Glu, Leu, Gly, Gly, Gly, Pro, Gly, Ala, Gly, Ser, Leu, Gln, Pro, Leu , Ala, Leu, Glu, Gly, Ser, Leu, Gln, Lys, Arg.

Chaotrope Hilfsmittel sind Verbindungen, die in wäßriger Lösung Wasserstoffbrückenbindungen brechen, beispielsweise Ammoniumsulfat, Guanidinhydrochlorid, Ethylencarbonat, Thiocyanat, Dimethylsulfoxid und Harnstoff.
Der Begriff hydrophobes Adsorberharz steht für nicht ionische, hydrophobe, vernetzte Polymere und/oder Copolymere, beispielsweise Polyacrylate oder Polystyrol oder Copolymere aus Styrol und Divinylbenzol, insbesondere polymere Adsorberharze mit großer Oberfläche und vielen großen Poren, z.B. Handelspräparate der Firmen Rohm and Haas oder Mitsubishi Chemical Industries Ltd. wie XAD16, XAD1600 oder HP20.
Unter dem Begriff Mercaptan werden Verbindungen verstanden, die wenigstens eine -SH-Gruppe enthalten. Bevorzugt sind wasserlösliche Mercaptane. Beispiele für diese Verbindungen sind Dithiothreitol, Dithioerythritol, 2-Mercaptoethanol, Cystein, Glutathion, Methylthioglykolat, 3-Mercapto-1,2-propandiol und 3-Mercaptopropionsäure.

Das Protein der Formel II kann mit Hilfe einer Vielzahl gentechnischer Konstrukte in Mikroorganismen gebildet werden (EP 0 489 780, EP 0 347 781, EP 0 453 969). Die gentechnischen Konstrukte werden in Mikroorganismen wie Escherichia coli oder Streptomyceten während der Fermentation exprimiert. Die gebildeten Proteine werden im Inneren der Mikroorganismen abgelagert (EP 0 489 780) oder in die Fermentationslösung ausgeschieden.

Für das erfindungsgemäße Verfahren können Proteine der Formel II eingesetzt werden, die direkt nach dem Zellaufschluß noch mit einer Vielzahl von Proteinen, die aus der Fermentationslösung und aus den Mikroorganismen stammen, verunreinigt sind. Die Proteine der Formel II können aber auch in vorgereinigter Form beispielsweise nach einer Fällung oder einer chromatographischen Reinigung eingesetzt werden.

Beim Verfahrensschritt A) geht man wie folgt vor:
Die Proteine werden in einem chaotropen Hilfsmittel oder in Mischungen von verschiedenen chaotropen Hilfsmitteln gelöst. Bevorzugt wird Harnstoff oder Guanidinhydrochlorid in einer Konzentration von 6 bis 9 M, bevorzugt 8 M, bezogen auf Wasser als Lösungsmittel, verwendet. Der pH-Wert des Reaktionsgemisches beträgt 8,5 bis 10,8. Beispiele für brauchbare Puffer sind Phosphat-, Tri(hydroxymethyl)aminomethan(Tris)-, Borat- oder Glycinpuffer. Die Konzentration der Puffersubstanzen im wäßrigem Medium beträgt bis zu 0,5 M, bevorzugt von 0,005 M bis 0,5 M, besonders bevorzugt von 0,05 bis 0,1 M. Anschließend wird die Proteinmischung mit einer wäßrigen Mercaptanlösung gemischt. Dadurch werden folgende Konzentrationen in der Reaktionslösung (bezogen auf Wasser) eingestellt:

Die Konzentration des Proteins der Formel II beträgt 0,05 bis 0,6 g/l, bevorzugt 0,1 bis 0,3 g/l. Die Menge des Mercaptans beträgt 2 bis 10 Reste -SH des Mercaptans pro Cysteinrest des Proteins der Formel II, bevorzugt 6 bis 9.
Der pH-Wert der Lösung beträgt 10 bis 11, bevorzugt 10,8. Es werden die obengenannten Pufferkomponenten verwendet. Der genaue pH-Wert wird durch Zugabe von Natronlauge eingestellt. Die Konzentration der Pufferkomponenten beträgt 0,005 bis 0,5 M, bevorzugt 0,05 bis 0,1 M.

Die Konzentration des chaotropen Hilfsmittels im Reaktionsansatz mit dem Mercaptan beträgt weniger als 1 M, bevorzugt 0,1 bis 0,8 M, insbesondere 0,3 bis 0,6 M. Als Mercaptan wird bevorzugt Cystein oder 2-Mercaptoethanol allein oder als Mischung eingesetzt.

Die Temperatur während der Faltung im Verfahrensschritt A) beträgt 0° C bis 50 °C, vorzugsweise 2° C bis 30 °C, insbesondere 4 °C bis 10 °C. Die Reaktionszeit beträgt 3 bis 12 Stunden, bevorzugt 4 bis 8 Stunden, insbesondere 5 Stunden.

Das Ergebnis von Verfahrensschritt A) ist ein Proinsulin, welches korrekt verbundene Cystinbrücken enthält.

Bei Verfahrensschritt B) wird die Reaktionslösung aus Verfahrensschritt A) auf einen pH-Wert von 6,5 bis 9, bevorzugt 8 bis 9, eingestellt. Trypsin oder ein trypsinähnliches Enzym wird in einer Menge von 1 bis 3 mg bezogen auf 3 g Proinsulin mit korrekt verbundenen Cystinbrücken dazugegeben. Gegebenenfalls wird Carboxypeptidase B in einer Menge von 3 bis 8 Einheiten bezogen auf 1 g Insulin der Formel I dazugegeben. Es kann auch eine Mischung von Trypsin und Carboxypeptidase B zugegeben werden. Die erhaltene Lösung wird nun von 4 bis 16 Stunden bei einer Temperatur von 4 °C bis 15 °C gerührt. Das Ergebnis von Verfahrensschritt B) ist ein Insulin der Formel I, welches korrekt verbundene Cystinbrücken enthält.

Bei Verfahrensschritt C) wird die Reaktionslösung aus Verfahrensschritt B) mit einer Säure wie Salzsäure oder Schwefelsäure auf einen pH-Wert von 2,5 bis 4,5 eingestellt. Wenn es zu Trübungen der Lösung kommen sollte, werden diese gegebenenfalls durch Filtration oder Zentrifugation entfernt. Die verbliebene Lösung wird mit einem hydrophoben Adsorberharz versetzt. Als günstig haben sich Harze vom Typ des XAD oder HP20 bewährt. Die Menge des Harzes beträgt 3 bis 50 g pro Liter Reaktionslösung, bevorzugt 20 bis 40 g/l.

Die erhaltene Suspension kann mit einem Salz wie NaCl bis zu einer Konzentration von 0,1 M versetzt werden, anschließend wird 3 bis 4 Stunden gerührt. Die Adsorption des Insulins der Formel I an das Harz wird durch Probennahme und Hochdruckflüssigchromatographie-Analyse (HPLC-Analyse) kontrolliert. Sobald kein Insulin mehr in der Lösung nachweisbar ist, wird das Adsorberharz von der wäßrigen Reaktionslösung abgetrennt (Verfahrensschritt D). Dies erfolgt beispielsweise durch Filtration oder Zentrifugation nach bekannten Methoden. Das Harz mit adsorbiertem Insulin der Formel I wird mit einer rein wäßrigen oder pufferhaltigen wäßrigen Lösung gewaschen. Insbesondere wird so lange gewaschen bis in der Waschlösung die Leitfähigkeit weniger als 0,4 mS/cm beträgt.

Die Desorption (Verfahrensschritt E) des Insulins der Formel I erfolgt nach bekannten Methoden, die vom verwendeten hydrophoben Adsorberharz abhängen. Die Desorption erfolgt bei XAD- oder HP20-Adsorberharzen beispielsweise durch eine wäßrige Lösung, die 20 bis 65 % eines mit Wasser mischbaren, nichtionischen organischen Lösungsmittels, z.B. ein (C₁-C₆)-Alkanol, enthält. Bevorzugt wird 35 % Isopropanol oder n-Propanol im Lösungsmittel Wasser verwendet. Die Desorption des Insulins der Formel I erfolgt beispielsweise durch Waschen mit der Isopropanollösung. Das Waschen kann auf einer Rührdrucknutsche durch Mischen mit der Isopropanollösung erfolgen oder durch Chromatographie in einer Säule. Das Volumenverhältnis Harz zu Isopropanollösung beträgt 1:1 bis 1:10, bevorzugt 1:1,1 bis 1:2. Die Waschschritte können ein- bis fünfmal wiederholt werden, bevorzugt zweimal. Die vereinigten Isopropanolfraktionen können direkt oder nach Verdünnung mit Wasser für weitere Chromatographiereinigungen verwendet werden.

In den folgenden Beispielen wird das erfindungsgemäße Verfahren im einzelnen beschrieben. Prozentangaben beziehen sich auf das Gewicht, sofern nicht anders angegeben.

### Beispiel 1

Durch Fermentation von genetisch modifizierten Escherichia-coli-Zellen (EP 0 489 780) wird ein Fusionsprotein mit folgender Aminosäuresequenz hergestellt.
Proinsulinsequenz 1 (Seq Id No. 4):

Met Ala Thr Thr Ser Thr Gly Asn Ser Ala Arg Phe Val Asn Gln His
Leu Cys Gly Ser His Leu Val Glu Ala Leu Tyr Leu Val Cys Gly Glu
Arg Gly Phe Phe Tyr Thr Pro Lys Thr Arg Arg Glu Ala Glu Asp Leu
Gln Val Gly Gln Val Glu Leu Gly Gly Gly Pro Gly Ala Gly Ser Leu
Gln Pro Leu Ala Leu Glu Gly Ser Leu Gln Lys Arg Gly Ile Val Glu
Gln Cys Cys Thr Ser Ile Cys Ser Leu Tyr Gln Leu Glu Asn Tyr Cys
Gly

Proinsulinsequenz 1 entspricht der Formel II, dabei ist
- X: C-Peptid von Humaninsulin,
- Y: Thr (B30),
- R¹: Phe (B1),
- R²: ein Peptid mit 11 Aminosäureresten,
- R³: Gly (A21) und
A2 - A20 die Aminosäuresequenz der A-Kette von Humaninsulin (Aminosäurereste 2 bis 20) und B2 - B29 die Aminosäuresequenz der B-Kette von Humaninsulin (Aminosäurereste 2 bis 29).

In den E.-coli-Zellen sammelt sich das exprimierte Fusionsprotein mit der Proinsulinsequenz 1 an und bildet Einschlußkörper. Nach Beendigung der Fermentation werden die Zellen durch Zentrifugation abgetrennt und durch übliche Hochdruckhomogenisation aufgeschlossen. Die freigesetzten Fusionsproteineinschlußkörperchen werden durch Zentrifugation isoliert.

2600 g der isolierten Fusionsproteineinschlußkörper (bezogen auf Trockenmasse nach Gefriertrocknung) mit der Proinsulinsequenz 1 werden in 200 l einer 8 M Guanidinhydrochlorid-Lösung bei pH 10,8 gelöst. Gegebenenfalls wird nach Zentrifugation geringer Mengen Trübstoffe die klare Lösung in 1800 l einer wäßrigen Cysteinlösung (250 g Cysteinhydrochloridhydrat) bei einem pH-Wert von 10,8 und einer Temperatur von 4 °C eingerührt. Der Anteil des insulinhaltigen Fusionproteins wird mit Hilfe von SDS-Elektrophorese-Scan (U.K. Lämmli, Nature, Band 227, Seiten 680-685, 1970) bestimmt. Er beträgt 45 %. Nach Abschluß der Faltungsreaktion wird mit Hilfe der analytischen HPLC der Gehalt an Proinsulinsequenz I mit korrekt verbundenen Cystinbrücken im Reaktionsansatz mit 760 g bestimmt.

Die 2000 l Lösung wird mit 6N HCl auf einen pH von 8,5 eingestellt. Dann erfolgt die Zugabe von 500 mg Trypsin. Es entsteht 305 g Insulin 2 nach HPLC-Messung.
Insulin 2 entspricht der Formel I, dabei ist
- Y: Thr (B30),
- Z: Arg-Arg,
- R¹: Phe (B1),
- R³: Gly (A21) und
A2 - A20 die Aminosäuresequenz der A-Kette von Humaninsulin (aminosäurereste 2 bis 20) und B2-B29 die Aminosäuresequenz der B-Kette von Humaninsulin (Aminosäurereste 2 bis 29).

Insulin 2 besteht aus der Seq Id No: 6 und 9, die über korrekt gebundene Cystinbrücken miteinander verbunden sind.

Die Lösung wird mit 6N HCl auf den pH-Wert von 3,5 eingestellt und der Leitfähigkeitswert mit Hilfe von gesättigter NaCl-Lösung auf etwa 10 mS/cm eingestellt. Eine leichte Trübung wird durch Zentrifugation entfernt. Zur klaren Lösung werden 75 kg von HP20 (Mitsubishi Chemical Industries Ltd., Düsseldorf, Deutschland) gegeben. Die Suspension wird etwa 16 Stunden bei 4°C langsam gerührt, bis kein Insulin 2 mehr im Überstand nachzuweisen ist. Durch Filtration über eine Nutsche wird das Harz abgetrennt und mit Wasser gewaschen, bis die Leitfähigkeit in der Waschlösung weniger als 0,4 mS/cm beträgt. Die Desorption des Produktes erfolgt durch Aufschlämmung des Harzes in 75 l 50%iger, wäßriger Isopropanollösung. Filtration und Inkubation mit der Isopropanollösung wird zweimal wiederholt. Nach 60 Minuten wird das Harz filtriert und anschließend mehrere Male in einer 35%igen Isopropanollösung inkubiert und gefiltert. Die Ausbeute an Insulin 2 beträgt 288 g.

Die Eluate, die Insulin 2 enthalten, können nach Verdünnung mit Wasser und pH-Einstellung unmittelbar auf einer Chromatographiesäule weiter gereinigt werden.

### HPLC-Analyse

0,5 g Protein werden in 40 ml einer Lösung aus 6 M Guanidinhydrochlorid, 50 mM Tris, pH 8,5, 5 mM Ethylendiamintetraacetat (EDTA), 1 % 2-Mercaptoethanol, 10 mM Dithiothreitol bei 95 °C für 2 min gelöst und dann bei 14000 g für 20 min zentrifugiert. Von dem klaren Überstand werden 0,02 ml auf eine Hochdruckflüssigchromatographiesäule aufgetragen.
- Säule:: ®Nucleogel RP 300-5/46 (Macherey & Nagel, Aachen, Deutschland)
- Gradient:: Puffer A: 0,1 % Trifluoressigsäure (TFA) Puffer B: 0,09 % TFA in Acetonitril
- Temperatur:: 55 °C
- Gesamtlaufzeit:: 40 min,
Der Gradient ist gekennzeichnet durch die folgende Mengen von Puffer B nach den entsprechenden Laufzeiten :
10 min 25%, 12 min 60 %, 13 min 90 %, 15 min 100 %.
- Fluß:: 1 ml/min
- Detektion:: 215 nm
- Retentionszeit von Insulin:: etwa 19 min

### Beispiel 2

Durch Fermentation von einem genetisch modifizierten E. coli (EP 0 347 781) wird ein Fusionsprotein mit folgender Aminosäuresequenz hergestellt: Fusionsprotein 3 (Seq Id No: 5)

Met Ala Pro Thr Ser Ser Ser Thr Lys Lys Thr Gln Leu Gln Leu Glu
His Leu Leu Leu Asp Leu Gln Met Ile Leu Asn Gly Ile Asn Asn Tyr
Lys Asn Pro Lys Leu Thr Arg Met Ile Glu Gly Arg Phe Val Asn Gln
His Leu Cys Gly Ser His Leu Val Glu Ala Leu Tyr Leu Val Cys Gly
Glu Arg Gly Phe Phe Tyr Thr Pro Lys Thr Arg Gly Ile Val Glu Gln
Cys Cys Thr Ser Ile Cys Ser Leu Tyr Gln Leu Glu Asn Tyr Cys Asn

Dieses Fusionsprotein enthält die Aminosäuresequenz von Proinsulin 3. In den E.-coli-Zellen sammelt sich das exprimierte Fusionsprotein 3 und bildet Einschlußkörper. Der Aufschluß der Zellen erfolgt wie in Beispiel 1. Die so erhaltenen Fusionsproteine 3 werden einer Bromcyanspaltung unterworfen, und es entsteht Proinsulin 3 (Seq Id No. 8).
Proinsulin 3 entspricht der Formel II, dabei ist
- X: Arg,
- Y: Thr (B30),
- R¹: Phe (B1),
- R²: ein Peptid mit 4 Aminosäureresten,
- R³: Asn (A21) und
A2 - A20 und B2 - B29 entsprechen der Aminosäuresequenz der A- und B-Ketten von Humaninsulin.

Nach der Bromcyanspaltung wird der Gehalt an Proinsulin 3 mit Hilfe der SDS-Elektrophorese in einer gefriergetrockneten Probe mit 9,2 % insulinhaltigem Protein quantitativ bestimmt.
2000 g von Proinsulin 3 werden wie im Beispiel 1 mit Cysteinhydrochloridhydrat inkubiert. Danach wird mittels analytische HPLC ein Gehalt von 1260 g an Proinsulin 3 mit korrekt verbundenen Cystinbrücken in dem gesamten Reaktionsansatz bestimmt. Wie in Beispiel 1 wird das Produkt mit 500 mg Trypsin behandelt.

Es entsteht das Insulin 4. Insulin 4 entspricht der Formel I, dabei ist
- Y: Thr (B30),
- Z: Arg,
- R¹: Phe (B1),
- R³: Asn (A21) und
A2 - A20 und B2 - B29 entsprechen der Aminosäuresequenz der A- und B-Ketten von Humaninsulin.
Insulin 4 besteht aus der Seq Id No: 1 und 7, die über korrekt gebundene Cystinbrücken miteinander verbunden sind.
Die resultierende Lösung (2 m³) enthält 800 g des Insulinderivates und wird mit 75 kg HP20 wie in Beispiel 1 behandelt. Nach Adsorption und Desorption enthält das vereinigte Eluat 703 g Insulin 4 (88 % Ausbeute).

## Patentansprüche

1. Verfahren zur Gewinnung von Insulin der Formel I 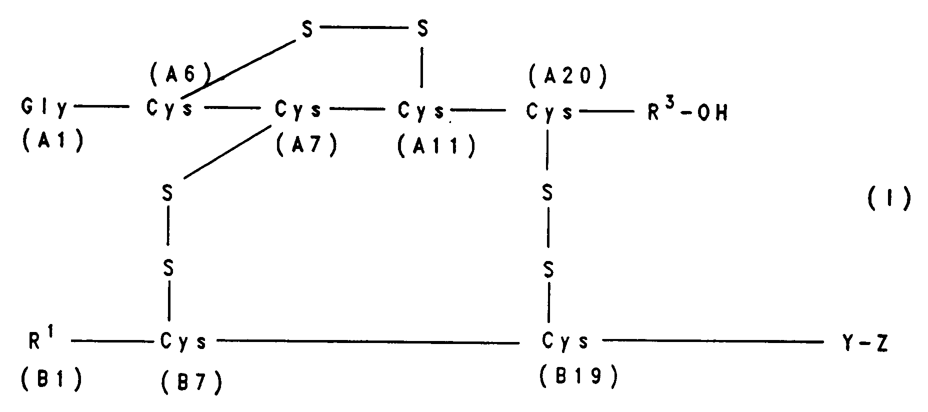 dadurch gekennzeichnet, daß man
A) ein Protein der Formel II,
R²-R¹-B2-B29-Y-X-Gly-A2-A20-R³ (II)
mit einer Menge eines Mercaptans, die 2 bis 10 Reste -SH des Mercaptans pro Cysteinrest des Proteins der Formel II ergibt, in Gegenwart von mindestens einem chaotropen Hilfsstoff in einem wäßrigen Medium bei einem pH-Wert von 10 bis 11 und einer Konzentration des Proteins der Formel II von 0,05 bis 0,3 g pro Liter wäßrigen Mediums umsetzt und
B) das erhaltene Proinsulin mit korrekt verbundenen Cystinbrücken mit Trypsin, einem trypsinähnlichen Enzym und gegebenfalls zusätzlich mit Carboxypeptidase B oder einer Mischung der genannten Enzyme zu dem Insulin der Formel I mit korrekt verbundenen Cystinbrücken umsetzt,
C) das so erhaltene Umsetzungsprodukt mit 3 bis 50 g eines hydrophoben Adsorberharzes pro Liter wäßrigen Mediums bei einem pH-Wert von 4 bis 7 versetzt,
D) das Adsorberharz mit adsorbiertem Insulin der Formel I isoliert und
E) das Insulin der Formel I vom Adsorberharz desorbiert; dabei ist in Formeln I und II
X
a) ein Aminosäurerest aus der Gruppe Lys oder Arg oder
b) ein Peptid mit 2 bis 35 Aminosäureresten, enthaltend den Aminosäurerest Arg oder Lys am N-terminalen- und Carboxylende des Peptids
Y ein genetisch kodierbarer Aminosäurerest,
Z
a) ein Aminosäurerest aus der Gruppe Lys oder Arg,
b) ein Peptid mit 2 oder 3 Aminosäureresten, enthaltend den Aminosäurerest Arg oder Lys am Carboxylende des Peptids, oder
c) OH,
R¹ ein Phenylalaninrest oder eine kovalente Bindung,
R²
a) ein Wasserstoffatom,
b) ein Aminosäurerest aus der Gruppe Lys oder Arg oder
c) ein Peptid mit 2 bis 45 Aminosäureresten, enthaltend den Aminosäurerest Arg oder Lys am Carboxylende des Peptids,
R³ ein genetisch kodierbarer Aminosäurerest und
die Reste A2 - A20 entsprechen der Aminosäuresequenz der A-Kette von Humaninsulin, tierischen Insulin oder einem Insulinderivat und
die Reste B2 - B 29 entsprechen der Aminosäuresequenz der B-Kette von Humaninsulin, tierischen Insulin oder einem Insulinderivat.

2. Verfahren nach Anspruch 1, dadurch gekennzeichnet, daß ein Protein der Formel II eingesetzt wird, dabei ist
X der Aminosäurerest Arg oder ein Peptid mit der Aminosäuresequenz der C-Kette von Humaninsulin,
Y ein Aminosäurerest aus der Gruppe Ala, Thr oder Ser,
R¹ der Aminosäurerest Phe,
R²
a) Wasserstoffatom oder
b) ein Peptid mit 2 bis 25 Aminosäureresten, enthaltend Arg am Carboxylende des Peptids,
R³ ein Aminosäurerest aus der Gruppe Asn, Ser oder Gly,
und die Reste A2 - A20 und B2 - B29 entsprechen der Aminosäuresequenz der A- und B-Ketten von Humaninsulin und es entsteht ein Insulin der Formel I mit korrekt verbundenen Cystinbrücken, dabei haben Y, R¹, R², R³, A2-A20 und B2-B29 die obengenannte Definition und
Z ist der Aminosäurerest Arg, der Peptidrest Arg-Arg oder OH.

3. Verfahren nach Anspruch 1, dadurch gekennzeichnet, daß ein Protein der Formel II eingesetzt wird, dabei ist
X der Aminosäurerest Arg oder ein Peptid mit 2 bis 35 Aminosäurereste, wobei am Anfang und Ende des Peptids zwei basische Aminosäurereste, insbesondere Arg und/oder Lys stehen,
Y der Aminosäurerest Thr,
R¹ der Aminosäurerest Phe,
R²
a) Wasserstoffatom oder
b) ein Peptid mit 2 bis 15 Aminosäureresten, an dessen Carboxylende der Aminosäurerest Arg steht,
R³ der Aminosäurerest Gly oder Asn,
und die Reste A2 - A20 und B2 - B29 entsprechen der Aminosäuresequenz der A- und B-Ketten von Humaninsulin und es entsteht ein Insulin der Formel I mit korrekt verbundenen Cystinbrücken, dabei haben Y, R¹, R², R³, A2-A20 und B2-B29 die obengenannte Definition und
Z ist der Aminosäurerest Arg, der Peptidrest Arg-Arg oder Lys-Lys oder OH.

4. Verfahren nach einem oder mehreren der Ansprüche 1 bis 3, dadurch gekennzeichnet, daß als Mercaptan Cystein oder Cysteinhydrochloridhydrat eingesetzt wird.

5. Verfahren nach einem oder mehreren der Ansprüche 1 bis 4, dadurch gekennzeichnet, daß als hydrophobes Adsorberharz ein vernetztes Polystyrol, Polyacrylat oder ein Copolymer aus Polystyrol und Divinylbenzyl eingesetzt wird.

6. Verfahren nach einem oder mehreren der Ansprüche 1 bis 5, dadurch gekennzeichnet, daß das Insulin der Formel I vom Adsorberharz mit Isopropanol oder n-Propanol desorbiert wird.

7. Verfahren nach einem oder mehreren der Ansprüche 1 bis 6, dadurch gekennzeichnet, daß im Verfahrensschritt C) 0,01 bis 0,1 M Salz zugefügt wird.

## Claims

1. A process for obtaining insulin of the formula I 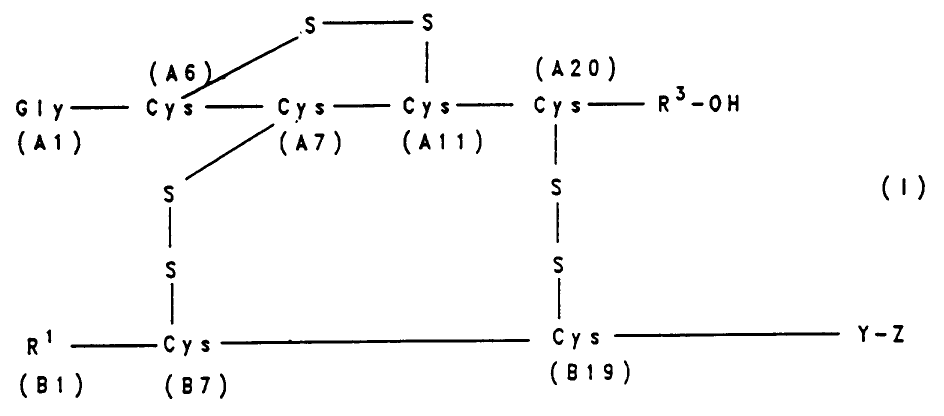 which comprises
A) reacting a protein of the formula II
R²-R¹-B2-B29-Y-X-Gly-A2-A20-R³ (II)
with an amount of a mercaptan which yields 2 to 10 -SH radicals of the mercaptan per cysteine residue of the protein of the formula II, in the presence of at least one chaotropic auxiliary in an aqueous medium at a pH of 10 to 11 and a concentration of the protein of the formula II of 0.05 to 0.3 g per liter of aqueous medium and
B) reacting the resulting proinsulin having correctly linked cystine bridges with trypsin, a trypsin-like enzyme and optionally additionally with carboxypeptidase B or a mixture of the enzymes mentioned to give the insulin of the formula I having correctly linked cystine bridges,
C) treating the reaction product thus obtained with 3 to 50 g of a hydrophobic adsorber resin per liter of aqueous medium at a pH of 4 to 7,
D) isolating the adsorber resin containing adsorbed insulin of the formula I and
E) desorbing the insulin of the formula I from the adsorber resin;
in this case, in formulae I and II
X is
a) an amino acid residue from the group consisting of Lys and Arg or
b) a peptide having 2 to 35 amino acid residues, containing the amino acid residue Arg or Lys at the N-terminal and carboxyl end of the peptide,
Y is a genetically encodable amino acid residue,
Z is
a) an amino acid residue from the group consisting of Lys and Arg,
b) a peptide having 2 or 3 amino acid residues, containing the amino acid residue Arg or Lys at the carboxyl end of the peptide or
c) OH,
R¹ is a phenylalanine residue or a covalent bond,
R² is
a) a hydrogen atom,
b) an amino acid residue from the group consisting of Lys and Arg or
c) a peptide having 2 to 45 amino acid residues, containing the amino acid residue Arg or Lys at the carboxyl end of the peptide,
R³ is a genetically encodable amino acid residue and
the residues A2 - A20 correspond to the amino acid sequence of the A chain of human insulin, animal insulin or an insulin derivative and
the residues B2 - B29 correspond to the amino acid sequence of the B chain of human insulin, animal insulin or an insulin derivative.

2. The process as claimed in claim 1, wherein a protein of the formula II is employed, in this case
X is the amino acid residue Arg or a peptide having the amino acid sequence of the C chain of human insulin,
Y is an amino acid residue from the group consisting of Ala, Thr and Ser,
R¹ is the amino acid residue Phe,
R² is
a) a hydrogen atom or
b) a peptide having 2 to 25 amino acid residues, containing Arg at the carboxyl end of the peptide,
R³ is an amino acid residue from the group consisting of Asn, Ser and Gly,
and the residues A2 - A20 and B2 - B29 correspond to the amino acid sequence of the A and B chains of human insulin and
an insulin of the formula I having correctly linked cystine bridges is formed, in this case Y, R¹, R², R³, A2-A20 and B2-B29 have the abovementioned definition and
Z is the amino acid residue Arg, the peptide residue Arg-Arg or OH.

3. The process as claimed in claim 1, wherein a protein of the formula II is employed, in this case
X is the amino acid residue Arg or a peptide having 2 to 35 amino acid residues, two basic amino acid residues, in particular Arg and/or Lys, being at the start and end of the peptide,
Y is the amino acid residue Thr,
R¹ is the amino acid residue Phe,
R² is
a) a hydrogen atom or
b) a peptide having 2 to 15 amino acid residues, at whose carboxyl end is the amino acid residue Arg,
R³ is the amino acid residue Gly or Asn, and the residues A2 - A20 and B2 - B29 correspond to the amino acid sequence of the A and B chains of human insulin and
an insulin of the formula I having correctly linked cystine bridges is formed, in this case Y, R¹, R², R³, A2-A20 and B2-B29 have the abovementioned definition and
Z is the amino acid residue Arg, the peptide residue Arg-Arg or Lys-Lys or OH.

4. The process as claimed in one or more of claims 1 to 3, wherein cysteine or cysteine hydrochloride hydrate is employed as the mercaptan.

5. The process as claimed in one or more of claims 1 to 4, wherein a crosslinked polystyrene, polyacrylate or a copolymer of polystyrene and divinylbenzene is employed as the hydrophobic adsorber resin.

6. The process as claimed in one or more of claims 1 to 5, wherein the insulin of the formula I is desorbed from the adsorber resin using isopropanol or n-propanol.

7. The process as claimed in one or more of claims 1 to 6, wherein 0.01 to 0.1 M salt is added in the process step C).

## Revendications

1. Procédé pour l'obtention d'insuline de formule I 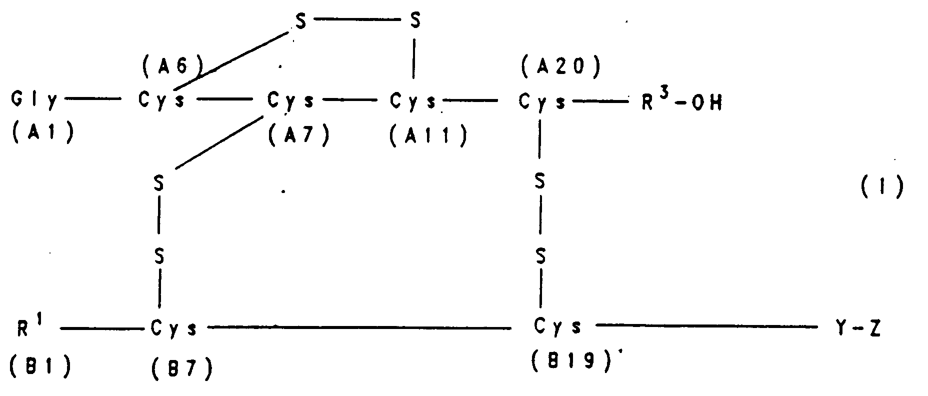 caractérisé en ce que
A) on fait réagir une protéine de formule II
R²-R¹-B2-B29-Y-X-Gly-A2-A20-R³ (II)
avec une quantité d'un mercaptan qui donne de 2 à 10 restes -SH du mercaptan par résidu cystéine de la protéine de formule II, en présence d'au moins un adjuvant chaotrope, dans un milieu aqueux, à un pH de 10 à 11 et à une concentration de la protéine de formule II allant de 0,05 à 0,3 g par litre de milieu aqueux, et
B) on fait réagir la pro-insuline obtenue, à ponts cystine correctement placés, avec de la trypsine, une enzyme similaire à la trypsine et éventuellement en outre avec de la carboxypeptidase B ou un mélange des enzymes citées, pour aboutir à l'insuline de formule I à ponts cystine correctement placés,
C) on ajoute au produit de réaction ainsi obtenu de 3 à 50 g d'une résine adsorbante hydrophobe, par litre de milieu aqueux, à un pH de 4 à 7,
D) on isole la résine adsorbante avec l'insuline de formule I adsorbée, et
E) l'insuline de formule I est désorbée de la résine adsorbante;
dans les formules I et II
X représentant
a) un résidu aminoacide choisi parmi Lys et Arg, ou
b) un peptide ayant de 2 à 35 résidus aminoacide, contenant le résidu aminoacide Arg ou Lys à l'extrémité N-terminale et l'extrémité carboxy-terminale du peptide,
Y représentant un résidu aminoacide génétiquement codable,
Z représentant
a) un résidu aminoacide choisi parmi Arg et Lys,
b) un peptide comportant 2 ou 3 résidus aminoacide, contenant le résidu aminoacide Arg ou Lys à l'extrémité carboxy-terminale du peptide, ou
c) OH,
R¹ représentant un résidu phénylalanine ou une liaison covalente,
R² représentant
a) un atome d'hydrogène,
b) un résidu aminoacide choisi parmi Lys et Arg, ou
c) un peptide ayant de 2 à 45 résidus aminoacide, contenant le résidu aminoacide Lys ou Arg à l'extrémité carboxy-terminale du peptide,
R³ représentant un résidu aminoacide génétiquement codable et
les restes A2-A20 correspondant à la séquence d'aminoacides de la chaîne A de l'insuline humaine, d'une insuline animale ou d'un dérivé d'insuline, et
les restes B2-B29 correspondant à la séquence d'aminoacides de la chaîne B de l'insuline humaine, d'une insuline animale ou d'un dérivé d'insuline.

2. Procédé selon la revendication 1, caractérisé en ce que l'on utilise une protéine de formule II dans laquelle
X est le résidu aminoacide Arg ou un peptide ayant la séquence d'aminoacides de la chaîne C de l'insuline humaine,
Y est un résidu aminoacide choisi parmi Ala, Thr ou Ser,
R¹ est le résidu aminoacide Phe,
R² représente
a) un atome d'hydrogène ou
b) un peptide ayant de 2 à 25 résidus aminoacide, contenant Arg à l'extrémité carboxy-terminale du peptide,
R³ est un résidu aminoacide choisi parmi Asn, Ser et Gly, et les restes A2-A20 et B2-B29 correspondent à la séquence d'aminoacides des chaînes A et B de l'insuline humaine, et
il en résulte une insuline de formule I à ponts cystine correctement placés, dans laquelle Y, R¹, R², R³, A2-A20 et B2-B29 ont les définitions données ci-dessus et
Z est le résidu aminoacide Arg, le résidu peptidique Arg-Arg ou OH.

3. Procédé selon la revendication 1, caractérisé en ce que l'on utilise une protéine de formule II dans laquelle
X est le résidu aminoacide Arg ou un peptide ayant de 2 à 35 résidus aminoacide, deux résidus aminoacide basiques, en particulier Arg et/ou Lys, se trouvant au début et à la fin du peptide,
Y est le résidu aminoacide Thr,
R¹ est le résidu aminoacide Phe,
R² représente
a) un atome d'hydrogène ou
b) un peptide ayant de 2 à 15 résidus aminoacide, à l'extrémité carboxy-terminale duquel se trouve le résidu aminoacide Arg,
R³ est le résidu aminoacide Gly ou Asn,
et les restes A2-A20 et B2-B29 correspondent à la séquence d'aminoacides des chaînes A et B de l'insuline humaine, et
il en résulte une insuline de formule I à ponts cystine correctement placés, dans laquelle Y, R¹, R², R³, A2-A20 et B2-B29 ont les définitions données plus haut, et
Z est le résidu aminoacide Arg, le reste peptidique Arg-Arg ou Lys-Lys ou OH.

4. Procédé selon une ou plusieurs des revendications 1 à 3, caractérisé en ce que l'on utilise en tant que mercaptan de la cystéine ou du chlorhydrate de cystéine hydraté.

5. Procédé selon une ou plusieurs des revendications 1 à 4, caractérisé en ce que l'on utilise en tant que résine adsorbante hydrophobe un polystyrène réticulé, un polyacrylate réticulé ou un copolymère de polystyrène et divinylbenzyle.

6. Procédé selon une ou plusieurs des revendications 1 à 5, caractérisé en ce que l'insuline de formule I est désorbée de la résine adsorbante à l'aide d'isopropanol ou de n-propanol.

7. Procédé selon une ou plusieurs des revendications 1 à 6, caractérisé en ce que, dans l'étape C) du procédé, on ajoute un sel à une concentration de 0,01 à 0,1 M.


SEQUENZPROTOKOLL

(1) ALGEMEINE INFORMATION:

(i) ANMELDER:
(A) NAME: Hoechst Aktiengesellschaft
(B) STRASSE: -
(C) ORT: Frankfurt am Main
(E) LAND: Federal Republic of Germany
(F) POSTLEITZAHL: 65926
(G) TELEPHON: 069-305-6047
(H) TELEFAX: 069-35-7175
(I) TELEX: 041234-700 hod

(ii) ANMELDETITEL: Verfahren zur Gewinnung von Insulin mit
korrekt verbundenen Cystinbrcken

(iii) ANZAHL DER SEQUENZEN: 9

(iv) COMPUTER-LESBARE FORM:
(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.25 (EPA)

(2) INFORMATION ZU SEQ ID NO: 1:

(i) SEQUENZ CHARAKTERISTIKA:
(A) LÄNGE: 21 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..21

(xi) SEQUENZBESCHREIBUNG: SEQ ID NO: 1:

Gly Ile Val Glu Gln Cys Cys Thr Ser Ile Cys Ser Leu Tyr Gln Leu
1               5                   10                  15

Glu Asn Tyr Cys Asn
            20

(2) INFORMATION ZU SEQ ID NO: 2:

(i) SEQUENZ CHARAKTERISTIKA:
(A) LÄNGE: 30 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..30

(xi) SEQUENZBESCHREIBUNG: SEQ ID NO: 2:

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr
            20                  25                  30

(2) INFORMATION ZU SEQ ID NO: 3:

(i) SEQUENZ CHARAKTERISTIKA:
(A) LÄNGE: 35 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..35

(xi) SEQUENZBESCHREIBUNG: SEQ ID NO: 3:

Arg Arg Glu Ala Glu Asp Leu Gln Val Gly Gln Val Glu Leu Gly Gly
1               5                   10                  15

Gly Pro Gly Ala Gly Ser Leu Gln Pro Leu Ala Leu Glu Gly Ser Leu
            20                  25                  30

Gln Lys Arg
        35

(2) INFORMATION ZU SEQ ID NO: 4:

(i) SEQUENZ CHARAKTERISTIKA:
(A) LÄNGE: 97 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..97

(xi) SEQUENZBESCHREIBUNG: SEQ ID NO: 4:

Met Ala Thr Thr Ser Thr Gly Asn Ser Ala Arg Phe Val Asn Gln His
1               5                   10                  15

Leu Cys Gly Ser His Leu Val Glu Ala Leu Tyr Leu Val Cys Gly Glu
            20                  25                  30

Arg Gly Phe Phe Tyr Thr Pro Lys Thr Arg Arg Glu Ala Glu Asp Leu
        35                  40                  45

Gln Val Gly Gln Val Glu Leu Gly Gly Gly Pro Gly Ala Gly Ser Leu
    50                  55                  60

Gln Pro Leu Ala Leu Glu Gly Ser Leu Gln Lys Arg Gly Ile Val Glu
65                  70                  75                  80

Gln Cys Cys Thr Ser Ile Cys Ser Leu Tyr Gln Leu Glu Asn Tyr Cys
                85                  90                  95

Gly

(2) INFORMATION ZU SEQ ID NO: 5:

(i) SEQUENZ CHARAKTERISTIKA:
(A) LÄNGE: 96 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..96

(xi) SEQUENZBESCHREIBUNG: SEQ ID NO: 5:

Met Ala Pro Thr Ser Ser Ser Thr Lys Lys Thr Gln Leu Gln Leu Glu
1               5                   10                  15

His Leu Leu Leu Asp Leu Gln Met Ile Leu Asn Gly Ile Asn Asn Tyr
            20                  25                  30

Lys Asn Pro Lys Leu Thr Arg Met Ile Glu Gly Arg Phe Val Asn Gln
        35                  40                  45

His Leu Cys Gly Ser His Leu Val Glu Ala Leu Tyr Leu Val Cys Gly
    50                  55                  60

Glu Arg Gly Phe Phe Tyr Thr Pro Lys Thr Arg Gly Ile Val Glu Gln
65                  70                  75                  80

Cys Cys Thr Ser Ile Cys Ser Leu Tyr Gln Leu Glu Asn Tyr Cys Asn
                85                  90                  95

(2) INFORMATION ZU SEQ ID NO: 6:

(i) SEQUENZ CHARAKTERISTIKA:
(A) LÄNGE: 32 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..32

(xi) SEQUENZBESCHREIBUNG: SEQ ID NO: 6:

Phe Val Asn Gln His Leu Cys Gly Ser His Leu Val Glu Ala Leu Tyr

1                   5                   10                  15

Leu Val Cys Gly Glu Arg Gly Phe Phe Tyr Thr Pro Lys Thr Arg Arg
            20                  25                  30

(2) INFORMATION ZU SEQ ID NO: 7:

(i) SEQUENZ CHARAKTERISTIKA:
(A) LÄNGE: 31 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..31

(xi) SEQUENZBESCHREIBUNG: SEQ ID NO: 7:

Phe Val Asn Gln His Leu Cys Gly Ser His Leu Val Glu Ala Leu Tyr
1               5                   10                  15

Leu Val Cys Gly Glu Arg Gly Phe Phe Tyr Thr Pro Lys Thr Arg
            20                  25                  30

(2) INFORMATION ZU SEQ ID NO: 8:

(i) SEQUENZ CHARAKTERISTIKA:
(A) LÄNGE: 56 Aminosäuren
(B) ART: Aminosäure
(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..56

(xi) SEQUENZBESCHREIBUNG: SEQ ID NO: 8:

Ile Glu Gly Arg Phe Val Asn Gln His Leu Cys Gly Ser His Leu Val
1               5                   10                  15

Glu Ala Leu Tyr Leu Val Cys Gly Glu Arg Gly Phe Phe Tyr Thr Pro
            20                  25                  30

Lys Thr Arg Gly Ile Val Glu Gln Cys Cys Thr Ser Ile Cys Ser Leu
        35                  40                  45

Tyr Gln Leu Glu Asn Tyr Cys Asn
    50                  55

(2) INFORMATION ZU SEQ ID NO: 9:

(i) SEQUENZ CHARAKTERISTIKA:
(A) LÄNGE: 21 Aminosäuren
(B) ART: Aminosäure

(C) STRANGFORM: Einzel
(D) TOPOLOGIE: linear

(ii) ART DES MOLEKÜLS: Protein

(vi) URSPRÜNLICHE HERKUNFT:
(A) ORGANISMUS: Escherichia coli

(ix) MERKMALE:
(A) NAME/SCHLÜSSEL: Protein
(B) LAGE: 1..21

(xi) SEQUENZBESCHREIBUNG: SEQ ID NO: 9:

Gly Ile Val Glu Gln Cys Cys Thr Ser Ile Cys Ser Leu Tyr Gln Leu
1               5                   10                  15

Glu Asn Tyr Cys Gly
            20